# EUROPEAN PATENT APPLICATION

(11) **EP 3 499 514 A1**
(43) Date of publication of application: **19.06.2019**
(21) Application number: 17207409.8
(22) Date of filing: 14.12.2017
(51) Int. Cl.: G16H 50/50, G16H 50/70, G16H 20/40, G16H 30/00

(54) **IMAGE-BASED AND DEMOGRAPHIC PREDICTION-BASED IMPLANT SIZE DETERMINATION**

(71) Applicant: Brainlab Ltd., 4951132 Petach-Tikva (IL)
(72) Inventor: Mackey, Marc, 4951132 Petach-Tikva (IL); Zenvirt, Ron, 4951132 Petach-Tikva (IL); Grushkevitz, Stav, 4951132 Petach-Tikva (IL)
(74) Representative: SSM Sandmair

(57) **Abstract**

Disclosed is a computer-implemented method of determining the size of an implant to be implanted at the location of an anatomical body part of a patient which encompasses determining the size of an implant, using both an image-based and a statistics-based approach. The image-based approach includes identifying, in a patient image, the position of the anatomical structure to be replaced by the implant, and appropriately planning the position and orientation of the implant in relation to surrounding anatomical structures. The statistic-based approach includes predicting the size of the implant from data describing properties of the patient, such as the patient's gender, ethnicity, weight, height, age etc. Depending on the properties of the patient and the outcome of the image analysis, both approaches may end with different data sets to be stored for later use in a picture archiving and communication system.

## Description

### FIELD OF THE INVENTION

The present invention relates to a computer-implemented method for determining the size of an implant to be implanted at the location of an anatomical body part of a patient, a corresponding computer program, a non-transitory program storage medium storing such a program and a computer for executing the program, as well as a medical system comprising an electronic data storage device and the aforementioned computer.

### TECHNICAL BACKGROUND

The present invention can be used for implant planning procedures e.g. in connection with a system for picture archiving and communication such as Quentry®, a product of Brainlab AG.

Aspects of the present invention, examples and exemplary steps and their embodiments are disclosed in the following. Different exemplary features of the invention can be combined in accordance with the invention wherever technically expedient and feasible.

### EXEMPLARY SHORT DESCRIPTION OF THE INVENTION

In the following, a short description of the specific features of the present invention is given which shall not be understood to limit the invention only to the features or a combination of the features described in this section.

The disclosed method encompasses determining the size of an implant, using both an image-based and a statistics-based approach. The image-based approach includes identifying, in a patient image, the position of the anatomical structure to be replaced by the implant, and appropriately planning the position and orientation of the implant in relation to surrounding anatomical structures. The statistic-based approach includes predicting the size of the implant from data describing properties of the patient, such as the patient's gender, ethnicity, weight, height, age etc. Depending on the properties of the patient and the outcome of the image analysis, both approaches may end with different data sets to be stored for later use in a picture archiving and communication system.

### GENERAL DESCRIPTION OF THE INVENTION

In this section, a description of the general features of the present invention is given for example by referring to possible embodiments of the invention.

In general, the invention reaches the aforementioned object by providing, in a first aspect, a computer-implemented medical method of determining the size of an implant to be implanted at least substantially at the location (for example, in lieu) of an anatomical body part of a patient. The anatomical body part in examples may include a bony tissue and/or cartilage structure such as at least part of a hip (i.e. a hip joint) or the at least part of a knee (i.e. a knee joint). The method is constituted to be constituted and/or comprises executing, on at least one processor of at least one computer (for example at least one computer being part of surgical planning system), the following exemplary steps which are executed by the at least one processor.

In a (for example first) exemplary step, anatomical body part data is acquired which describes an identity of the anatomical body part. For example, the anatomical body part data comprises information identifying the anatomical body part. Such information may take the form of a numeric variable, character or string indicating that the anatomical body part is for example the knee or the hip. This information may be read from a surgical plan as predetermined information or entered manually by a person planning a surgical implantation procedure (e.g. a surgeon) for replacing the anatomical body part by an implant.

In a (for example second) exemplary step, metadata about the patient is acquired which describes at least one of anatomical or physiological parameters of the patient or at least one other patient-related parameter. The metadata about the patient describes at least one of the following:
- the patient's name;
- the patient's gender;
- the patient's body mass index;
- the patient's ethnicity;
- the date and time of envisaged surgery to be carried out on the patient;
- a hospital at which surgery is to be carried out on the patient;
- the name of a surgeon who is to carry out surgery on the patient;
- the patient's weight; or
- the patient's height.

The metadata about the patient may be read from a surgical plan as predetermined information or entered manually by a person planning a surgical implantation procedure (e.g. a surgeon) for replacing the anatomical body part by an implant.

In a (for example third) exemplary step, patient image data is acquired which describes an image of the anatomical body part and a calibration device. In examples, the patient image data has been generated by application of an x-ray-based or magnetic resonance-based imaging modality or other tomographic or non-tomographic imaging modality to at least the anatomical body part and the calibration device. The calibration device is placed at least substantially at the position of the anatomical body part when the imaging is conducted for generating the patient image data. The calibration device comprises for example metal spheres (made of e.g. stainless steel) of predetermined (i.e. at least one of known or fixed) geometry such as diameter (e.g. 1 inch and/or 2.54 cm) such as those included in in the VoyantMark or KingMark® (a product of Brainlab AG) orthopaedic calibration device. The size of the image depiction of the calibration device can be used in conjunction with the predetermined geometry to re-scale the size of the image depiction of the anatomical body part to the actual size of the anatomical body part so as to eliminate the influence of the imaging geometry on the depicted size of the anatomical body part.

In a (for example fourth) exemplary step, the patient image data is analysed for the presence of an image depiction of the calibration device and it is determined whether the image depiction of the calibration device has been detected. For example, this may include segmentation of the image for the presence of at least one image feature corresponding to a predetermined image depiction of the calibration device. If it is determined that the image depiction of the calibration device has been detected, the image of the anatomical body part and the calibration device is automatically calibrated, for example re-scaled according to the predetermined geometry of the calibration device as described above. Then, the image is analysed for the presence of an image depiction of the anatomical body part and it is determined whether the image depiction of the anatomical body part has been detected. This may be done by applying for example at least one of the following two approaches either separately or in combination: the first approach encompasses comprises grey-level based analysis of the image to determine at least one image feature corresponding to a predetermined grey-level depiction of the anatomical body part; the second approach encompasses acquiring atlas data describing a generic image-based representation of the anatomical body part, and comparing the atlas data to the patient image data to determine the position of the anatomical body part in the image (for example by applying an image fusion algorithm, for example rigid or elastic fusion, to the two data sets).

If it is determined that at least part of the image depiction of the anatomical body part has been detected in the image, the method continues with a (for example fifth) exemplary step of automatically determining, based on the anatomical body part data and the patient image data and the determined image depictions of the calibration device and the at least part of the anatomical body part, implant planning data describing an at least partial planning (for example, automatic planning) of the implant in the patient's body including the information about the size of the implant. If only an image depiction of less than the complete anatomical body part has been detected in the image, the method may conduct a partial planning (for example, automatic planning) for only that part of the anatomical body part, the depiction of which has been detected in the image. The planning encompasses establishing a spatial relationship (e.g. at least one of position or orientation) between the geometry (i.e. a dataset describing at least the outline) of the implant and the anatomical structures representing the immediate surroundings of the anatomical body part to be replaced by the implant. In other words, the planning encompasses fitting a graphical object corresponding to the implant into the anatomical structures in lieu of the anatomical body part, and accordingly sizing and/or dimensioning the implant. An example of a tool usable for such a planning is the TraumaCad® orthopaedic preoperative planning and templating solution, a product of Brainlab AG.

In a subsequent (for example sixth) exemplary step, the planning image data and the patient image data and the metadata about the patient are then saved in association with each other, for example in a picture archiving and communication system (PACS) and/or an application for accessing a PACS such as Quentry®, a product of Brainlab AG.

If, however, it is determined that not at least part of the image depiction of the anatomical body part has been detected in the image, the method continues with a (for example seventh) exemplary step of saving (for example, only) the patient image data and the metadata about the patient in association with each other (and not saving any planning image data), for example in a PACS or application for accessing a PACS. This is done for example without any prior execution of automatic planning.

If it is determined that the image depiction of the calibration device has not been detected, the method continues with a (for example eighth) exemplary step of determining, based on metainformation describing properties of the patient image data, whether an image sizing of the image is available. The metainformation is generally included in digital data files containing medical image information such as the patient image data. For example, the DICOM (digital imaging and communications in medicine) data format includes a definition for such metainformation. The properties described by the metainformation include for example information describing a sizing, for example pixel size and/or spacing, of the image elements (pixels and/or voxels). Thus, the eighth exemplary step encompasses whether such information is available from the metainformation.

If it is determined that the image sizing is available, the method continues with a (for example ninth) exemplary step of re-scaling the size of image features depicted in the image according to the image sizing, which may also be called oversizing the image, so that the size of the image depiction of the anatomical body part conforms better to the actual size of the anatomical body part. Then, the method continues with executing the above-described step of determining whether at least part of the image depiction of the anatomical body part has been detected in the image. If, however, it is determined that the image sizing is not available (from the metainformation), the method continues with the above-described for example seventh exemplary step.

Additionally to the above and unconditionally, the method executes a (for example tenth) exemplary step of analysing the metadata about the patient as to whether it contains information about the body-mass index (and/or information about at least the patient's height and the patient's weight) so that the patient's body-mass index can be calculated online of the patient. If it is determined that the metadata about the contains information about the body-mass index of the patient (and/or information about at least the patient's height and the patient's weight so that the patient's body-mass index can be calculated online), it is then determined in a (for example eleventh) exemplary step whether a demographic prediction for the implant size is available from a predetermined dataset (such as an existing database) containing information about the probability of the implant size in dependence on the metadata about the patient. This determination can also be based on information contained in the metadata about the patient, such as at least one of the patient's gender, ethnicity or age.

If it is determined that the demographic prediction for the implant size is available from the predetermined dataset, the implant size is determined to be the implant size associated with a predetermined probability in dependence on the metadata about the patient. Then, the implant size and the metadata about the patient and the patient image data are saved in a (for example twelfth) exemplary step in association with each other, for example in a PACS or an application for accessing a PACS. If, however, it is determined that the metadata about the does not contain information about the body-mass index of the patient or that the demographic prediction for the implant size is not available from the predetermined dataset, the method continues with the (for example seventh) exemplary step.

An example of the method according to the first aspect encompasses a step of acquiring atlas data describing a generic image-based representation of the anatomical body part and for example information describing the relevant medical indication (e.g. the envisaged procedure, for example implantation procedure). Then the size of the implant may then be determined based on (e.g. from) the atlas data. For example, the size of the implant is determined by determining the size of the anatomical body part described by that atlas data, for example the size of the implant is set to be at least substantially the (if applicable, re-scaled) size of the description of the anatomical body part by the atlas data. Alternatively or additionally, the step of determining the size of the implant based on the atlas data may be or not be part of at least one of the for example fifth exemplary step of automatically determining the implant planning data or the step of determining the implant size to be the implant size associated with a predetermined probability in dependence on the metadata about the patient.

In a second aspect, the invention is directed to a computer program which, when running on at least one processor (for example, a processor) of at least one computer (for example, a computer) or when loaded into at least one memory (for example, a memory) of at least one computer (for example, a computer), causes the at least one computer to perform the above-described method according to the first aspect. The invention may alternatively or additionally relate to a (physical, for example electrical, for example technically generated) signal wave, for example a digital signal wave, carrying information which represents the program, for example the aforementioned program, which for example comprises code means which are adapted to perform any or all of the steps of the method according to the first aspect. A computer program stored on a disc is a data file, and when the file is read out and transmitted it becomes a data stream for example in the form of a (physical, for example electrical, for example technically generated) signal. The signal can be implemented as the signal wave which is described herein. For example, the signal, for example the signal wave is constituted to be transmitted via a computer network, for example LAN, WLAN, WAN, for example the internet. The invention according to the second aspect therefore may alternatively or additionally relate to a data stream representative of the aforementioned program.

In a third aspect, the invention is directed to a non-transitory computer-readable program storage medium on which the program according to the fourth aspect is stored.

In a fourth aspect, the invention is directed to at least one computer (for example, a computer), comprising at least one processor (for example, a processor) and at least one memory (for example, a memory), wherein the program according to the fourth aspect is running on the processor or is loaded into the memory, or wherein the at least one computer comprises the computer-readable program storage medium according to the fifth aspect.

In a fifth aspect, the invention is directed to a medical system (for example, a picture archiving and communication system), comprising:
a) the at least one computer according to the fourth aspect; and
b) at least one electronic data storage device storing at least one of the patient image data or at least part of the metadata about the patient,
wherein the at least one computer is operably coupled to the at least one electronic data storage device for acquiring, from the at least one data storage device, the at least one of the patient image data or the at least part of the metadata about the patient.

In an example of the system according to the fifth aspect, the medical system comprises a user input interface (such as at least one of a touchscreen, keyboard, or mouse) operably coupled to the at least one computer for entering at least part of the metadata about the patient.

In an example of the system according to the fifth aspect, execution of at least one of the for example sixth, seventh or twelfth exemplary steps causes the respective data (i.e. the applicable at least one of the metadata about the patient, patient image data or planning image data) to be saved in the at least one electronic data storage device For example, the invention does not involve or in particular comprise or encompass an invasive step which would represent a substantial physical interference with the body requiring professional medical expertise to be carried out and entailing a substantial health risk even when carried out with the required professional care and expertise.

For example, the invention does not comprise a step of inserting an implant into the patient's body. More particularly, the invention does not involve or in particular comprise or encompass any surgical or therapeutic activity. The invention is instead directed as applicable to calculating data describing the planned size of the implant. For this reason alone, no surgical or therapeutic activity and in particular no surgical or therapeutic step is necessitated or implied by carrying out the invention.

The present invention also relates to the use of the device/system or any embodiment thereof for planning a surgical implantation procedure. The use comprises for example execution of the method according to the fist aspect by the computer of the medical system according to the fifth aspect.

### DEFINITIONS

In this section, definitions for specific terminology used in this disclosure are offered which also form part of the present disclosure.

### Computer implemented method

The method in accordance with the invention is for example a computer implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (laaS). The cloud computer can function as a virtual host for an operating system and/or data processing application which is used to execute the method of the invention. The cloud computer is for example an elastic compute cloud (EC2) as provided by Amazon Web Services™. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. An example of such a digital lightbox is Buzz®, a product of Brainlab AG. The monitor may also be the monitor of a portable, for example handheld, device such as a smart phone or personal digital assistant or digital media player.

The invention also relates to a program which, when running on a computer, causes the computer to perform one or more or all of the method steps described herein and/or to a program storage medium on which the program is stored (in particular in a non-transitory form) and/or to a computer comprising said program storage medium and/or to a (physical, for example electrical, for example technically generated) signal wave, for example a digital signal wave, carrying information which represents the program, for example the aforementioned program, which for example comprises code means which are adapted to perform any or all of the method steps described herein.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer; a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

### Acquiring data

The expression "acquiring data" for example encompasses (within the framework of a computer implemented method) the scenario in which the data are determined by the computer implemented method or program. Determining data for example encompasses measuring physical quantities and transforming the measured values into data, for example digital data, and/or computing (and e.g. outputting) the data by means of a computer and for example within the framework of the method in accordance with the invention. The meaning of "acquiring data" also for example encompasses the scenario in which the data are received or retrieved by (e.g. input to) the computer implemented method or program, for example from another program, a previous method step or a data storage medium, for example for further processing by the computer implemented method or program. Generation of the data to be acquired may but need not be part of the method in accordance with the invention. The expression "acquiring data" can therefore also for example mean waiting to receive data and/or receiving the data. The received data can for example be inputted via an interface. The expression "acquiring data" can also mean that the computer implemented method or program performs steps in order to (actively) receive or retrieve the data from a data source, for instance a data storage medium (such as for example a ROM, RAM, database, hard drive, etc.), or via the interface (for instance, from another computer or a network). The data acquired by the disclosed method or device, respectively, may be acquired from a database located in a data storage device which is operably to a computer for data transfer between the database and the computer, for example from the database to the computer. The computer acquires the data for use as an input for steps of determining data. The determined data can be output again to the same or another database to be stored for later use. The database or database used for implementing the disclosed method can be located on network data storage device or a network server (for example, a cloud data storage device or a cloud server) or a local data storage device (such as a mass storage device operably connected to at least one computer executing the disclosed method). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, for example determining data, does not involve a surgical step and in particular does not involve a step of treating a human or animal body using surgery or therapy. In order to distinguish the different data used by the present method, the data are denoted (i.e. referred to) as "XY data" and the like and are defined in terms of the information which they describe, which is then preferably referred to as "XY information" and the like.

### Imaging geometry

The information on the imaging geometry preferably comprises information which allows the analysis image (e.g. x-ray image) to be calculated, given a known relative position between the imaging geometry analysis apparatus and the analysis object (anatomical body part) to be analysed by x-ray radiation, if the analysis object which is to be analysed is known, wherein "known" means that the spatial geometry (size and shape) of the analysis object is known. This means for example that three-dimensional, "spatially resolved" information concerning the interaction between the analysis object (anatomical body part) and the analysis radiation (x-ray radiation) is known, wherein "interaction" means for example that the analysis radiation is blocked or partially or completely allowed to pass by the analysis object. The location and in particular orientation of the imaging geometry is for example defined by the position of the x-ray device, for example by the position of the x-ray source and the x-ray detector and/or for example by the position of the multiplicity (manifold) of x-ray beams which pass through the analysis object and are detected by the x-ray detector. The imaging geometry for example describes the position (i.e. the location and in particular the orientation) and the shape (for example, a conical shape exhibiting a specific angle of inclination) of said multiplicity (manifold). The position can for example be represented by the position of an x-ray beam which passes through the centre of said multiplicity or by the position of a geometric object (such as a truncated cone) which represents the multiplicity (manifold) of x-ray beams. Information concerning the above-mentioned interaction is preferably known in three dimensions, for example from a three-dimensional CT, and describes the interaction in a spatially resolved way for points and/or regions of the analysis object, for example for all of the points and/or regions of the analysis object. Knowledge of the imaging geometry for example allows the location of a source of the radiation (for example, an x-ray source) to be calculated relative to an image plane (for example, the plane of an x-ray detector). With respect to the connection between three-dimensional analysis objects and two-dimensional analysis images as defined by the imaging geometry, reference is made for example to the following publications:
1. "An Efficient and Accurate Camera Calibration Technique for 3D Machine Vision", Roger Y. Tsai, Proceedings of the IEEE Conference on Computer Vision and Pattern Recognition. Miami Beach, Florida, 1986, pages 364-374
2. "A Versatile Camera Calibration Technique for High-Accuracy 3D Machine Vision Metrology Using Off-the-Shelf TV Cameras and Lenses", Roger Y. Tsai, IEEE Journal of Robotics and Automation, Volume RA-3, No. 4, August 1987, pages 323-344.
3. "Fluoroscopic X-ray Image Processing and Registration for Computer-Aided Orthopedic Surgery", Ziv Yaniv
4. EP 08 156 293.6
5. US 61/054,187

### Atlas / Atlas segmentation

Preferably, atlas data is acquired which describes (for example defines, more particularly represents and/or is) a general three-dimensional shape of the anatomical body part. The atlas data therefore represents an atlas of the anatomical body part. An atlas typically consists of a plurality of generic models of objects, wherein the generic models of the objects together form a complex structure. For example, the atlas constitutes a statistical model of a patient's body (for example, a part of the body) which has been generated from anatomic information gathered from a plurality of human bodies, for example from medical image data containing images of such human bodies. In principle, the atlas data therefore represents the result of a statistical analysis of such medical image data for a plurality of human bodies. This result can be output as an image - the atlas data therefore contains or is comparable to medical image data. Such a comparison can be carried out for example by applying an image fusion algorithm which conducts an image fusion between the atlas data and the medical image data. The result of the comparison can be a measure of similarity between the atlas data and the medical image data. The atlas data comprises image information (for example, positional image information) which can be matched (for example by applying an elastic or rigid image fusion algorithm) for example to image information (for example, positional image information) contained in medical image data so as to for example compare the atlas data to the medical image data in order to determine the position of anatomical structures in the medical image data which correspond to anatomical structures defined by the atlas data.

The human bodies, the anatomy of which serves as an input for generating the atlas data, advantageously share a common feature such as at least one of gender, age, ethnicity, body measurements (e.g. size and/or mass) and pathologic state. The anatomic information describes for example the anatomy of the human bodies and is extracted for example from medical image information about the human bodies. The atlas of a femur, for example, can comprise the head, the neck, the body, the greater trochanter, the lesser trochanter and the lower extremity as objects which together make up the complete structure. The atlas of a brain, for example, can comprise the telencephalon, the cerebellum, the diencephalon, the pons, the mesencephalon and the medulla as the objects which together make up the complex structure. One application of such an atlas is in the segmentation of medical images, in which the atlas is matched to medical image data, and the image data are compared with the matched atlas in order to assign a point (a pixel or voxel) of the image data to an object of the matched atlas, thereby segmenting the image data into objects.

For example, the atlas data includes information of the anatomical body part. This information is for example at least one of patient-specific, non-patient-specific, indication-specific or non-indication-specific. The atlas data therefore describes for example at least one of a patient-specific, non-patient-specific, indication-specific or non-indication-specific atlas. For example, the atlas data includes movement information indicating a degree of freedom of movement of the anatomical body part with respect to a given reference (e.g. another anatomical body part). For example, the atlas is a multimodal atlas which defines atlas information for a plurality of (i.e. at least two) imaging modalities and contains a mapping between the atlas information in different imaging modalities (for example, a mapping between all of the modalities) so that the atlas can be used for transforming medical image information from its image depiction in a first imaging modality into its image depiction in a second imaging modality which is different from the first imaging modality or to compare (for example, match or register) images of different imaging modality with one another.

### Imaging methods

In the field of medicine, imaging methods (also called imaging modalities and/or medical imaging modalities) are used to generate image data (for example, two-dimensional or three-dimensional image data) of anatomical structures (such as soft tissues, bones, organs, etc.) of the human body. The term "medical imaging methods" is understood to mean (advantageously apparatus-based) imaging methods (for example so-called medical imaging modalities and/or radiological imaging methods) such as for instance computed tomography (CT) and cone beam computed tomography (CBCT, such as volumetric CBCT), x-ray tomography, magnetic resonance tomography (MRT or MRI), conventional x-ray, sonography and/or ultrasound examinations, and positron emission tomography. For example, the medical imaging methods are performed by the analytical devices. Examples for medical imaging modalities applied by medical imaging methods are: X-ray radiography, magnetic resonance imaging, medical ultrasonography or ultrasound, endoscopy, elastography, tactile imaging, thermography, medical photography and nuclear medicine functional imaging techniques as positron emission tomography (PET) and Single-photon emission computed tomography (SPECT), as mentioned by Wikipedia.

The image data thus generated is also termed "medical imaging data". Analytical devices for example are used to generate the image data in apparatus-based imaging methods. The imaging methods are for example used for medical diagnostics, to analyse the anatomical body in order to generate images which are described by the image data. The imaging methods are also for example used to detect pathological changes in the human body. However, some of the changes in the anatomical structure, such as the pathological changes in the structures (tissue), may not be detectable and for example may not be visible in the images generated by the imaging methods. A tumour represents an example of a change in an anatomical structure. If the tumour grows, it may then be said to represent an expanded anatomical structure. This expanded anatomical structure may not be detectable; for example, only a part of the expanded anatomical structure may be detectable. Primary/high-grade brain tumours are for example usually visible on MRI scans when contrast agents are used to infiltrate the tumour. MRI scans represent an example of an imaging method. In the case of MRI scans of such brain tumours, the signal enhancement in the MRI images (due to the contrast agents infiltrating the tumour) is considered to represent the solid tumour mass. Thus, the tumour is detectable and for example discernible in the image generated by the imaging method. In addition to these tumours, referred to as "enhancing" tumours, it is thought that approximately 10% of brain tumours are not discernible on a scan and are for example not visible to a user looking at the images generated by the imaging method.

### Elastic fusion, image fusion/morphing, rigid

Image fusion can be elastic image fusion or rigid image fusion. In the case of rigid image fusion, the relative position between the pixels of a 2D image and/or voxels of a 3D image is fixed, while in the case of elastic image fusion, the relative positions are allowed to change.

In this application, the term "image morphing" is also used as an alternative to the term "elastic image fusion", but with the same meaning.

Elastic fusion transformations (for example, elastic image fusion transformations) are for example designed to enable a seamless transition from one dataset (for example a first dataset such as for example a first image) to another dataset (for example a second dataset such as for example a second image). The transformation is for example designed such that one of the first and second datasets (images) is deformed, for example in such a way that corresponding structures (for example, corresponding image elements) are arranged at the same position as in the other of the first and second images. The deformed (transformed) image which is transformed from one of the first and second images is for example as similar as possible to the other of the first and second images. Preferably, (numerical) optimisation algorithms are applied in order to find the transformation which results in an optimum degree of similarity. The degree of similarity is preferably measured by way of a measure of similarity (also referred to in the following as a "similarity measure"). The parameters of the optimisation algorithm are for example vectors of a deformation field. These vectors are determined by the optimisation algorithm in such a way as to result in an optimum degree of similarity. Thus, the optimum degree of similarity represents a condition, for example a constraint, for the optimisation algorithm. The bases of the vectors lie for example at voxel positions of one of the first and second images which is to be transformed, and the tips of the vectors lie at the corresponding voxel positions in the transformed image. A plurality of these vectors is preferably provided, for instance more than twenty or a hundred or a thousand or ten thousand, etc. Preferably, there are (other) constraints on the transformation (deformation), for example in order to avoid pathological deformations (for instance, all the voxels being shifted to the same position by the transformation). These constraints include for example the constraint that the transformation is regular, which for example means that a Jacobian determinant calculated from a matrix of the deformation field (for example, the vector field) is larger than zero, and also the constraint that the transformed (deformed) image is not self-intersecting and for example that the transformed (deformed) image does not comprise faults and/or ruptures. The constraints include for example the constraint that if a regular grid is transformed simultaneously with the image and in a corresponding manner, the grid is not allowed to interfold at any of its locations. The optimising problem is for example solved iteratively, for example by means of an optimisation algorithm which is for example a first-order optimisation algorithm, such as a gradient descent algorithm. Other examples of optimisation algorithms include optimisation algorithms which do not use derivations, such as the downhill simplex algorithm, or algorithms which use higherorder derivatives such as Newton-like algorithms. The optimisation algorithm preferably performs a local optimisation. If there is a plurality of local optima, global algorithms such as simulated annealing or generic algorithms can be used. In the case of linear optimisation problems, the simplex method can for instance be used.

In the steps of the optimisation algorithms, the voxels are for example shifted by a magnitude in a direction such that the degree of similarity is increased. This magnitude is preferably less than a predefined limit, for instance less than one tenth or one hundredth or one thousandth of the diameter of the image, and for example about equal to or less than the distance between neighbouring voxels. Large deformations can be implemented, for example due to a high number of (iteration) steps.

The determined elastic fusion transformation can for example be used to determine a degree of similarity (or similarity measure, see above) between the first and second datasets (first and second images). To this end, the deviation between the elastic fusion transformation and an identity transformation is determined. The degree of deviation can for instance be calculated by determining the difference between the determinant of the elastic fusion transformation and the identity transformation. The higher the deviation, the lower the similarity, hence the degree of deviation can be used to determine a measure of similarity.

A measure of similarity can for example be determined on the basis of a determined correlation between the first and second datasets.

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1: illustrates a flow diagram comprising the basic steps of the method according to the first aspect;
- Fig. 2: constitutes a legend for the flow diagram of Fig. 1; and
- Fig. 3: is a schematic illustration of the system according to the fifth aspect.

### DESCRIPTION OF EMBODIMENTS

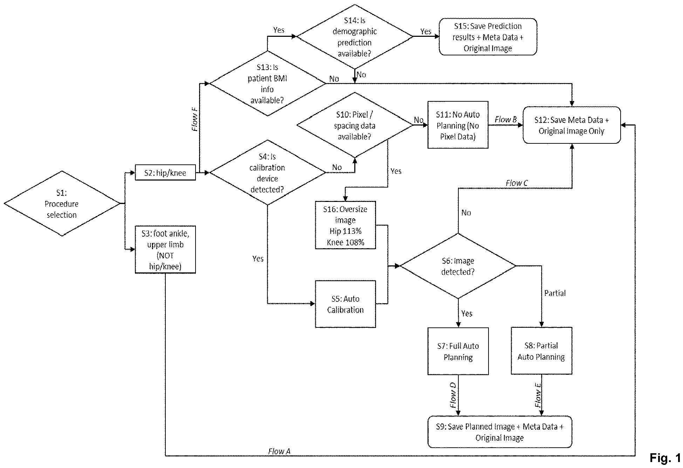

Figure 1 illustrates the basic steps of the method according to the first aspect, in which steps S1 and S2 encompass selection of the implant procedure and the type of anatomical body part and therefore acquisition of the anatomical body part data. If it turns out that the type of anatomical is the foot ankle or an upper limb (specifically, not the knee or hip), the method proceeds directly to step S12 which encompasses saving only the metadata about the patient and the patient image data without saving any planning image data. In step S4, the patient image data is analysed for the presence of an image depiction of the calibration device. If it is determined that the image depiction of the calibration is present (yes in step S4), the method p proceeds to performing automatic calibration of the patient image data in step S5 which encompasses a re-sizing of the image of the anatomical body part and the calibration device. The method then continues with step S6 which encompasses analysing the image for the presence (and, if applicable, position) of an image depiction of the anatomical body part which depending on the result of this analysis followed by partial auto planning of the implant in step S8 or full auto planning of the implant in step S7 (in both cases, if step S6 results in yes), or saving only the metadata about patient and the patient image data without saving an planning image data in step S12 (if step S6 results in no).

If step S4 results in that no image depiction of the calibration device is detected in the image, the method proceeds to step S10 which encompasses determining whether an image sizing of the image is available. If the result of this determination is positive (yes), the method continues with step S16 which encompasses the re-scaling of the image which in turn is followed by step S6. If the result of step 10 is, however, negative (no), the method does not execute any automatic planning (see the box marked by S11) and proceeds to step S12.

In addition to the above-described steps of Figure 1, the method comprises, after execution of steps S1 and S2, a step S13 of determining whether information about the body-mass index of the patient is available (e.g. from the patient metadata or another data source). If the result of this determination is positive (yes), the method proceeds with determining, in step S14, whether a demographic prediction of the implant size is available. If the answer to this this determination is positive (yes), the method proceeds with saving the result of the prediction (i.e. information defining the predicted size of the implant) in association with the metadata about the patient and the patient image data in step S15. If the result of step S13 or step S14 is negative, the method proceeds to above-described step S12.

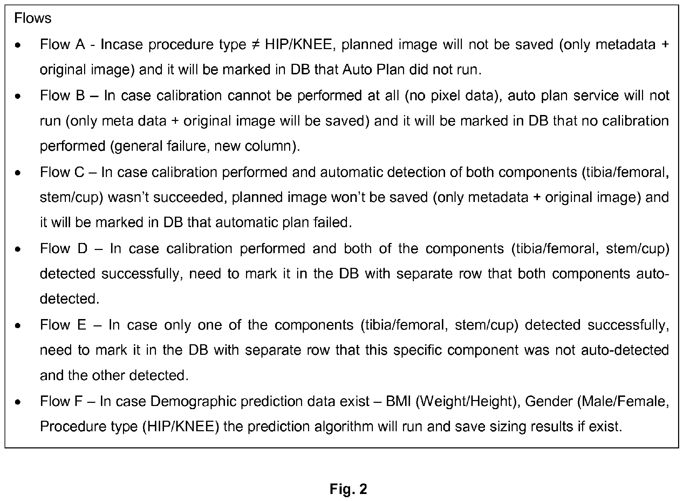

Figure 2 is a short legend to flows A to F defined in Figure 1. Details of these flows are also presented in below Table 1.

**Table 1**

| Workflow ID | Description | Data Field | Data Value | User Notifications | PACS Result/PACS accessing application result |
|---|---|---|---|---|---|
| **Flow A** | In case procedure type ≠ HIP/KNEE | Planning type | Inventory (Not Auto) | Procedure type not supporting image auto plan. | Planned image will not be saved - only meta data + original image. |
| | | Planning Result | Failed | | |
| | | | | | * Templates sizing based on Statistical Prediction only if available |
| **Flow B** | In case calibration cannot be performed at all (no pixel data), auto plan service won't run | Calibration | Calibration failure | Calibration couldn't be performed, image auto plan not created. | Planned image will not be saved |
| | | Planning type | Automatic (Auto Plan) | | - Only meta data + originalimage |
| | | Planning Result | Failed | | * Templates sizing based on Statistical Prediction only if available |
| **Flow C** | Calibration performed and automatic detection of both components (tibia/femoral, stem/cup) wasn't succeeded | Calibration | Calibration Type (Kingmark / ball / oversizing) | Automatic detection failed, image auto plan not created. | Planned image will not be saved |
| | | Planning type | Automatic (Auto Plan) | | - Only meta data + original image. |
| | | Planning Result | Failed (on both components) | | * Templates sizing based on Statistical Prediction only if available. |
| **Flow D** | Calibration performed and both of the components (tibia/femoral, stem/cup) detected successfully. | Calibration | Calibration Type (Kingmark / ball / oversizing) | Automatic planned image created successfully. | Planned image + meta data + original image will be saved. |
| | | Planning type | Automatic (Auto Plan) | | * Templates sizing based on the Automatic detection and Statistical Prediction (if available) |
| | | Planning Result | Succeeded (mark on both components auto detected). | | |
| **Flow E** | Calibration performed and only one of the components (tibia/femoral, stem/cup) automatic detected successfully. | Calibration | Calibration Type (Kingmark / ball / oversizing) | Automatic planned image created successfully. | Planned image + meta data + original image will be saved. |
| | | Planning type | Automatic (Auto Plan) | | * Templates sizing based on Automatic detections and Statistical Prediction (if available). |
| | | Planning Result | Succeeded (mark specific component wasn't auto detected and the other detected) | | |
| Statistical Prediction | Statistical prediction of Templates Sizing based on patient BMI will be saved in the DB. | Planning type | Calculation | ---- | Sizing of the templates will be saved (same as today - just with the identifier it came from the statistical prediction calculation |
| | Available only when user enter BMI, the templates exist / have the detection points. | | | | |

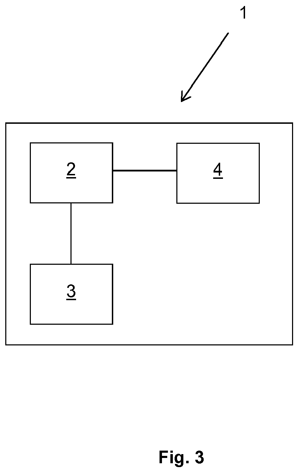

Figure 3 is a schematic illustration of the medical system 1 according to the fifth aspect. The system is in its entirety identified by reference sign 1 and comprises a computer 2, an electronic data storage device (such as a hard disc) 3 for storing at least the patient data and a user input interface 4. The components of the medical system 1 have the functionalities and properties explained above with regard to the fifth aspect of this disclosure.

Further details of the method according to the first aspect are described in the following.

### PACS for Auto Sizing Ortho - Basic Workflow

### General

Auto Sizing Ortho aims at providing automatic template sizing suggestions using an image detection algorithm and calculation based on patient BMI. Surgery data and patient information filled in manually via a web form also being uploaded as part of the case folder.

### Workflow

A user will view the image from the PACS or Q/R directly from the web form, then fill in details in the form (define data on the x-ray image, patient details and select templates) and submit. The entire data, including the original DICOM image, is uploaded to the PACS and/or an application for accessing a PACS such as Quentry® (a product of Brainlab AG) where it is automatically processed.

The user can easily export reports of the surgery information and required implants.

### 1.1. Login to the PACS accessing application

- With a user validly registered with the PACS accessing application, credentials (user ID and password), the login is performed via the gateway and not directly to the application.
- A web form session timeout is set per the user's setting in the PACS accessing application, the user must accept "keep me logged" in via checkbox otherwise re-login is required each time after submitting case.
- A basic error handling (wrong user name / password, empty fields entered, password expired) is included.

### 1.2.AutoSizer Ortho Web Form

A web form is used in order to manually enter the planning data:
The form will be part of the Quentry Gateway and will include the following
   - Images Selection (by Q/R via PACS or directly from the web form)
   - Planning information - Procedure, body side and Templates selection.
   - Patient information (the metadata about the patient) - BMI, race, surgery date, hospital and surgeon name. Data fields are included which are taken from the DICOM Patient Name/ ID, date of birth and gender.
   - Send to the PACS accessing application.
   - Basic error handling / user notifications are available.

### 1.2.1. Images Selection - URL Integration or Q/R

The solution may offer two alternatives for image selection:
1. PACS Integration - solution is generic as much as possible, to fit many PACS systems as well usability and easy to use.
   - Integration is by accession number and study ID, the form should show all query images in thumbnails
   - User selects the desired images to run auto-plan (the automatic planning procedure of steps S7 or, as applicable, step S8) on
   - Selection is up to two images
   - If only one or two images exist, the thumbnails will be auto-populated to the users in the form without the selection stage.
2. Free text search via the Web Form.
   - Once user run the URL it should trigger this free search mode.
   - It will be instead of the image selection window and will include free text bar
   - Three query levels are supported:
      ■ study - study description, study date, patient details (name, ID, date of birth, gender).
      ■ series - imaging modality, number of images, series description and date
      ■ instance level - No data is presented to the user on the Image level (thumbnails only)
   - User selects the desired images to run auto-plan on
   - Selection should be up to 2 images
      - Once done and submitting to Auto Plan user will get back to a clean form and can Q/R again.

### 1.2.2. Planning information *

■ Procedure: hip, knee, upper limb, foot ankle, trauma and spine
■ Side - selection: AP(anterior-posterior) or LAT (lateral)
■ Laterality - selection: left or light (one selection for both images)

### 1.2.3. Implants

■ Search - free text to search the required templates. Entering two letters automatically initiates the search.
■ Results - Show the search results with scroll (max top 15 results, user can narrow the search by typing more specific keywords).
■ Selection - select the templates:
   - For HIP/KNEE - only 2 templates can be added.
   - For all other procedures - multiple selections of templates.
   - Selected templates can be also removed after selection.

### 1.2.4. Patient details

■ Patient Details from DICOM file (all are read only fields)
   - Name
   - ID
   - DOB
   - Gender
■ BMI
   - Weight - User should choose units KG / LBS
   - Height - User should choose units CM / Feet
   - Default units - KG /CM
      Client should do the calculation and save it to Quentry DB in metric system units.
■ Race
   - Asian
   - Black
   - Indian
   - Latino
   - Mediterranean
   - Middle eastern
   - Native American
   - White
   - Unknown
   - Other

### 1.2.5. Surgery

- Surgery Date and Time (time/date picker)
- Hospital (Institution name, free text)
- Surgeon (name, free text)
- Comments (free text)

### 1.2.6. Sent to Plan

Once form is completed, user should "send to plan" to start auto-plan process.

### 1.3. Upload to the PACS accessing application

- Uploaded case should include the following info
   ■ The web form planning and patient information as was filled out by the user.
   ■ Metadata from the DICOM.
   ■ The DICOM images (the patient image data).
- Once the process completed (Gateway sent the data), the user should be notified on success or failure (The user still in the web form with hourglass until the process completed).
- User should have the option to Logout from the form.

### 1.4.AutoSizer Ortho - Automatic Planning Service

The automatic planning service should include these basic functionalities:
- Process the web form data.
- Image recognition for Auto templates placement and sizing.
- Store to the PACS accessing application - metadata (the metadata about the patient), planning data, sizing prediction and the planned image (the planning image data).
- Generate notifications of success/failures to the PACS accessing application and reports

The service is able to run as well in the PACS accessing application cloud and as a ground component.

### 1.4.1. Process the web form data

The data from the web form and the DICOM image should be transferred via the PACS accessing application gateway to the automatic planning service.

The data is submitted via a WCF protocol and the automatic process on the image begins using the provided data:
- Image defined data (mandatory fields)
   ∘ Procedure: HIP, KNEE, Trauma, Spine, Upper Limb or Foot Ankle.
   ∘ Orientation: Side (Left/Right) and Laterality (AP/LAT)
- Implants - The templates names (mandatory fields)
- General data - surgery date and time, Institution, comments, surgeon name.
- Patient data - patient name, ID, date of birth, gender (all the four are read only, taken from the DICOM), BMI and race (optional fields)

### 1.4.2. Image detection - Valid only for HIP and KNEE procedures.

By using the defined procedure, image orientation and the selected templates. This step contains 2 main functions:
- Calibration
   ∘ Automatic Calibration - The predefined device (KingMark® / ball) runs for automatic calibration by determining the magnification factor in the X-ray
   ∘ Oversize - In case automatic calibration marker device cannot be found in the image and there is pixel spacing, values will be set for HIP and knee (predefined configurable)
   ∘ No pixel spacing - calibration will be failed and planned image will not be created (save only the web form data entered and the original image).
- Automatic planning
   ∘ The planned outcome is templated image and sizing of the implants types (Stem/Cup, Tibia/Femoral).
   ∘ Trauma, Spine, Upper Limb or Foot Ankle - no calibration and image recognition, only the original DICOM file and web form data are uploaded to the PACS accessing application.
   ∘ Auto Planning is relevant only for HIP/KNEE procedures.
      HIP
      - Automatic suggestion of the templates sizing.
         Phase 2 -
      - Automatic position of the cup and stem templates (The cup is positioned in the area of the acetabulum and the stem is placed in the femur canal.)
      - Leg Length Discrepancy (LLD) - Draws a tangent line on the image by marking two points' at the most inferior points on the ischial tuberosities.
      KNEE
      - Automatic suggestion of the templates sizing
         Phase 2 -
      - Automatic position of the tibia and Femoral templates Resection Line - measures the size of the medial and lateral bone cuts, marks the femoral anatomical and mechanical axis and the tibial axis.
Note - Auto Alignment shouldn't be performed.

### Detection use cases:

∘ Partial automatic detection
   - in case of partial detection of one of the components the auto sizing will be set for the one which was detected while the second will not have value (no default value size).
   - Planned image will be created
∘ Image Automatic detection completely failed.
   - Only the web form data and the original image will be saved.
   - Templates sizing will not be inserted to the data base (no default values)
   - Planned image will not be created.

### 1.4.3. Store to the PACS accessing application

Once the process ends the results are being generated to the PACS accessing application.

There should be an option (via configuration) to save only the planning data without the planned image to the PACS accessing application (only the original DICOM will be saved not the templated planned DICOM)

The data should contain:
■ Original DICOM image
■ Metadata from the web form.
■ Planning data include implants properties and reports
■ Planned DICOM Image (phase 2 - having only the ability to enable feature).

### 1.4.4. Generate notifications of success/failures

The service triggers several notifications which will be presented to the user in the web portal and stored in the data base.
■ Failures will only be stored in DB for internal use, not sent to users.
   ∘ Calibration - calibration failed and planning cannot be performed.
   ∘ Auto detection - automatic detection for Hip / Knee cannot be performed or partially only on one of the implant components.
   ∘ Statistical Prediction - couldn't run due to missing data.
   ∘ General error - image couldn't be uploaded due to "system problems".
■ Success
   Any data uploaded to Quentry will be defined as "success", user will get one "ok" notification in the web portal and one email.
   Examples for success:
   ∘ Image successfully uploaded to folder
   ∘ Auto detection successfully run and stored implant sizes
   ∘ Prediction successfully run and predict sizing
   ∘ Web form data uploaded

### 1.5. Reports and Activity

The output includes the following data in addition:
- Planning Type (five types - Manual input, Auto detection, Statistical sizing, TraumaCad plan and Actual implant size - future support)
- Implant size - based on the Statistical Prediction where applicable.
- Surgery Time
- Surgeon Name
The following will be only in the DB stats report
- BMI - weight, height
- Race

### 1.6.AutoSizer Ortho - Predict Implant Sizing based on patient demographics

Statistical Algorithm that helps to predict HIP and KNEE implant sizing based on patient demographics. The sizing results should be stored in the Quentry DB and exported via the CSV report, as part of the automatic service that runs on the cloud.

The following data must be submitted in order the algorithm to run
- Procedure Type and Treated Side
- BMI - weight and height
- Gender - Female / Male
- Selected implants

The results will be stored as today under implant type and size
Not all templates are supported in order to generate the size results, in this case no value at all only template name.

## Claims

1. A computer-implemented medical method of determining the size of an implant to be implanted at the location of an anatomical body part of a patient, the method being constituted to be executed by a computer and comprising the following steps:
a) anatomical body part data is acquired (S1, S2) which describes an identity of the anatomical body part;
b) metadata about the patient is acquired which describes at least one of anatomical or physiological parameters of the patient or at least one other patient-related parameter;
c) patient image data is acquired which describes an image of the anatomical body part and a calibration device;
c1) the patient image data is analysed (S4) for the presence of an image depiction of the calibration device and it is determined whether the image depiction of the calibration device has been detected, and
if it is determined that the image depiction of the calibration device has been detected,
c11) the image of the anatomical body part and the calibration device is automatically calibrated (S5); and
c12) the image is analysed (S6) for the presence of an image depiction of the anatomical body part and it is determined whether the image depiction of the anatomical body part has been detected, and
if it is determined that at least part of the image depiction of the anatomical body part has been detected,
the method continues with
c121) automatically determining (S7, S8), based on the anatomical body part data and the patient image data and the determined image depictions of the calibration device and the at least part of the anatomical body part, implant planning data describing an at least partial planning of the implant in the patient's body including the information about the size of the implant; and
c122) saving (S9) the planning image data and the patient image data and the metadata about the patient in association with each other; and
if it is determined that not at least part of the image depiction of the anatomical body part has been detected,
c3) saving (S12) the patient image data and the metadata about the patient in association with each other;
if, in step c1), it is determined that the image depiction of the calibration device has not been detected,
c2) determining (S10), based on metainformation describing properties of the patient image data, whether an image sizing of the image is available, and
if it is determined that the image sizing is available,
the method continues with
c21) re-scaling (S16) the size of image features depicted in the image according to the image sizing, and then with
executing (S6), based on the resized image, step c12); and
if it is determined that the image sizing is not available,
the method continues with step c3) (S12);
d) the metadata about the patient is analysed (S13) whether it contains information about the body-mass index of the patient, and
if it is determined that the metadata about the contains information about the body-mass index of the patient,
d1) it is determined (S14) whether a demographic prediction for the implant size is available from a predetermined dataset containing information about the probability of the implant size in dependence on the metadata about the patient, and
if it is determined that the demographic prediction for the implant size is available from the predetermined dataset,
d2) the implant size is determined to be the implant size associated with a predetermined probability in dependence on the metadata about the patient; and
d3) the implant size and the metadata about the patient and the patient image data are saved (S15) in association with each other; and
if it is determined that the metadata about the does not contain information about the body-mass index of the patient or that the demographic prediction for the implant size is not available from the predetermined dataset,
the method continues with
executing step c3) (S12).

2. The method according to the preceding claim, comprising
e) acquiring atlas data describing a generic image-based representation of the anatomical body part,
f) wherein the size of the implant is determined based on the atlas data.

3. The method according to the preceding claim, wherein the size of the implant is determined in step f) by determining the size of the anatomical body described by the atlas data, for example by setting the size of the implant to be at least substantially the size of the anatomical body part described by the atlas data.

4. The method according to any one of the two immediately preceding claims, wherein at least step f) is part of at least one of steps c121) or d2).

5. The method according to any one of the two immediately preceding claims, wherein at least step f) is not part of at least one of steps c121) or d2).

6. The method according to any one of the preceding claims, wherein step c1) includes segmentation of the image for the presence of at least one image feature corresponding to a predetermined image depiction of the calibration device.

7. The method according to any one of the preceding claims, wherein step c12) comprises grey-level based analysis of the image to determine at least one image feature corresponding to a predetermined grey-level depiction of the anatomical body part.

8. The method according to any one of the preceding claims, comprising
acquiring atlas data describing a generic image-based representation of the anatomical body part,
wherein step c12) comprises comparing the atlas data to the patient image data to determine the position of the anatomical body part in the image.

9. The method according to any one of the preceding claim, wherein the metadata about the patient describes at least one of the following:
- the patient's name;
- the patient's gender;
- the patient's body mass index;
- the patient's ethnicity;
- the date and time of envisaged surgery to be carried out on the patient;
- a hospital at which surgery is to be carried out on the patient;
- the name of a surgeon who is to carry out surgery on the patient;
- the patient's weight; or
- the patient's height.

10. The method according to any one of the preceding claims, wherein the patient image data has been generated by application of an x-ray-based or magnetic resonance-based imaging modality or other tomographic or non-tomographic imaging modality to at least the anatomical body part and the calibration device.

11. A program which, when running on a computer or when loaded onto a computer, causes the computer to perform the method steps of the method according to any one of the preceding claims.

12. A program storage medium on which the program according to the preceding claim is stored for example in a non-transitory form;
and/or a computer comprising at least one processor and a memory and the program storage medium, wherein for example the program is running on the computer or loaded into the memory of the computer;
and/or a signal wave or a digital signal wave, carrying information which represents the program;
and/or a data stream which is representative of the program.

13. A medical system (1), comprising:
a) he at least one computer (2) according to the preceding claim; and
b) at least one electronic data storage device (3) storing at least one of the patient image data or at least part of the metadata about the patient,
wherein the at least one computer is operably coupled to the at least one electronic data storage device for acquiring, from the at least one data storage device (3), the at least one of the patient image data or the at least part of the metadata about the patient.

14. The system (1) according to the preceding claim, wherein further comprising
a user input interface (4) operably coupled to the at least one computer (2) for entering at least part of the metadata about the patient.

15. The system (1) according to any one of the two immediately preceding claims, wherein at least one of steps c122), c3) or d3) saves the respective data in the at least one electronic data storage device (3).
